# EUROPEAN PATENT APPLICATION

(11) **EP 1 832 656 A1**
(43) Date of publication of application: **12.09.2007**
(21) Application number: 07011876.5
(22) Date of filing: 31.05.2001
(51) Int. Cl.: C12N 15/31, C07K 14/37, C07K 16/14, A61K 39/00

(54) **Method for isolating and using fungal hemolysin**

(30) Priority: 01.06.2000 US 208301 P
(62) Divisional of application: 01946012.0
(71) Applicant: UNITED STATES ENVIRONMENTAL PROTECTION AGENCY, Washington, D.C. 20004 (US)
(72) Inventor: The designation of the inventor has not yet been filed
(74) Representative: Brown, David Leslie

(57) **Abstract**

Hemolysin isolated from hemolysin-producing fungi can be used to detect if a human or other animal has been exposed to a hemolysin-producing fungus. The method and proteins of the present invention can be used to screen humans and other animals for exposure to such fungi, as well as to produce vaccines for protecting humans and other animals that may be exposed to such fungi.

## Description

### Field of the Invention

The present invention relates to the use of fungal hemolysins for diagnostic analysis, environmental monitoring and medical applications.

### Cross-Reference to Related Applications

The present invention claims priority from provisional application Serial No. 60/208,301, filed June 1, 2000, the entire contents of which are hereby incorporated by reference.

### Background of the Invention

Exposure to fungi may have significant health implications. Fungi have now been found to cause conditions such as chronic sinusitis, asthma, and allergies. In addition, exposure to fungi has been correlated with conditions such as sick-building syndrome, infantile pulmonary hemorrhage, neurological disorders, and other related conditions. However, there has been no way to correlate the symptoms exhibited with exposure to fungi.

In particular, asthma rates in the United States and in many other parts of the world have nearly doubled. The number of asthma sufferers is now more than 17 million in the United States alone, with an estimated five million of them children. The death rate for children from asthma increased by 78% between 1980 and 1993. The cost of medical care for asthma, including hospitalization and treatment, exceeds $14 billion a year.

Sick building syndrome (SBS) is a complex condition that may involve many factors. However, the occurrence of fungi in buildings affected with SBS has been positively correlated with the condition.

The most common chronic disease in the United sates is sinusitis. It afflicts more than 37 million Americans. This sinus condition is characterized by inflammation of the membranes of the nose and sinus cavity. Symptoms can include runny nose, nasal congestion, headaches, and polyp growth in the sinus cavities.
Recently, the cause of this disease was found to be fungi.

In order to determine if one has been exposed, or is currently being exposed, to certain fungi, a marker is required. For many fungi, such as *Stachybotrys chartarum,* this is not possible because normal antibody production to the body or mycelium of the organism does not occur in humans. As noted above, many health conditions can be caused by exposure to fungi, and it is important to identify the cause of the condition so that appropriate treatment can be commenced.

Hemolysins are molecules that are designated as such because they have the ability to lyse red blood cells (RBC). It is now recognized that the biological significance of these toxins goes beyond their lysis of RBC to their more general ability to form pores in many cells (Bhakdi et al., J. Clin. Inves. 85:1746-1753, 1990). Currently the consensus is that the majority of relevant bacterial pathogens product pore forming proteins (Bhakdi et al., Arch. Microbiol. 165:73-79, 1996). Hemolysins have been isolated and purified from many bacterial pathogens, and they are generally important virulence factors (Bhakdi et al., 1996; Cavalieri et al., Microbiol. Rev. 48:326-342, 1984; Johnson et al., Infect. Immun. 49:765-769, 1985; O'Reilly et al., Microb. Pathog. 1:125-138, 1986; Ou Said et al., Am. J. Vet. Res. 49:1657-1660, 1988; Van Der Vijer et al., J. Med. Microbiol. 8:279-287, 1975; Welch et al., Nature (London) 294:665-667, 1981). However, there are no reports of isolating hemolysins from fungi.

It is believed that bacterial cytolysins act primarily by killing host cells, but non-lethal reactions in other cells, including endothelial and immune cells, occur as a result of exposure to these toxins (Emert et al., Lab. Invest. 66:362-369, 1993; Iwaki et al., Infect. Immun. 67:2763-2768, 1999). Many bacterial hemolysins create pores not only in RBC, but also in the membranes of nucleated cells, e.g., neutrophils, monocytes, and endothelial cells (Bhakdi et al., 1996; Grimminger et al., J. Clin. Invest 88:1531-1539, 1991; Seeger et al., Am. Rev. Resp. Dis. 143:785-797, 1991) and can affect aggregation of platelets (Iwaki et al., 1999). These hemolysin mediated responses affect the pathophysiology of the host.

Unfortunately, because normal antibody production to the body or myceluim of many fungi does not occur in humans, there is no method to quantify exposure to many fungi.

### Summary of the Invention

It is an object of the present invention to overcome the deficiencies in the prior art.

It is an object of the present invention to provide a method and reagent for screening humans and other animals for exposure to hemolysin-producing fungi.

It is another object of the present invention to provide a method and reagent for screening humans and other animals for exposure to *Stacybotrys chartarum.* .

It is another object of the present invention to isolate fungal hemolysins.

It is a further object of the present invention to identify strains of fungi using an in *vitro* test.

It is still another object of the present invention to provide a vaccine against fungal infections.

It is another object of the present invention to provide pharmaceuticals which create pores in membranes for a variety of uses.

It is still another object of the present invention to screen strains of fungi isolated from buildings, homes, schools, etc. to identify fungal strains which produce a hemolysin.

It is yet a further object of the present invention to provide anti-bacterial compositions comprising fungal hemolysin.

It is another object of the present invention to provide anti-fungal compositions comprising fungal hemolysin.

### Detailed Description of the Invention

The present inventor has discovered a method for purifying fungal hemolysin proteins so that these hemolysin proteins can be used to demonstrate exposure to fungi for environmental or medical evaluations, as well as to prepare vaccines against fungal infection and to produce anti-bacterial and anti-fungal compositions. These fungal hemolysin proteins may be present in the blood, urine, saliva, or other measurable body fluid or material of a human or animal infected with the fungi. Antibodies produced by any appropriate technique, such as in rabbits, or monoclonal antibodies, against the hemolysin protein are used to assay for these proteins. The assay itself can be of any conventional immunoassay type, such as ELISA, RIA, etc.

Fungal hemolysins can also be used in anti-bacterial and anti-fungal compositions in a suitable carrier. These fungal hemolysins produce pores in the bacterial or fungal cells, destroying the cells.

The hemolysin protein can also be used in pharmaceuticals in suitable amounts for producing pores in cell membranes, which is particularly useful in delivering drugs across the blood-brain barrier. The fungal hemolysins can be used to selectively kill certain types of cells, such as (rapidly growing) cancer cells. Additionally, hemolysins have been found to alter immune functions, and fungal hemolysins can be used in much the same way as bacterial hemolysins.

By growing strains of hemolysin producing fungi in vitro and isolating the hemolysin, it is now possible to use the protein obtained to identify fungi which are isolated from buildings, homes, schools, and the like. The fungal strains are grown in a conventional synthetic medium such as tryptic soy broth, at about 37°C.

### Isolation of Fungal hemolysin

*S. chartarum* conidia were used to inoculate 500 ml of tryptic soy broth (TSB) (Becton Dickinson, Sparks, MD) in a one liter flask placed onto an incubator-shaker (LabLine Inc., Melrose Park, IL) set at 36 ± 1°C and mixed at 200 rpm/min. After seven days of incubation, the cells and debris were removed from the culture by centrifuging for 15 minutes at 5000x g in a RC5 centrifuge (Dupont Instruments, Newton, CT), and the supernatant was recovered. The supernatant was centrifuged in a Millipore Centricon plus 80 filter apparatus with a MW cutoff of 50 kDa (Millipore, Bedford, MA) at 4000 x g for 15 minutes in a RC5 centrifuge. The concentrate was recovered according to the manufacturer's instructions.

The concentrate was subjected to gel filtration using Sephadex G 100-50 (Sigma, St. Louis MO), hydrated in 0.2M sodium azide for five days, and giving a final bed of 0.5 x 14 cm. The concentrate was added to the top of the gel filtration column using 0.2M sodium azide as solvent. Fractions of 0.25 ml were collected at 1.5 ml per hour using a fraction collector (ISCO, Lincoln, NE). Then, 10 microliters of each reaction was plated onto sheep's blood agar (SBA) (Becton Dickinson, Sparks, MD) and incubated at 37°C and hemolysis noted.

The homiletical active fractions were combined and isolated twice using the D-SALT™ Polyacrylamide 6000 desalting column (Pierce, Rockford, IL). The final desalted solution was frozen at -80°C and lyophilized using a Spin Vac (Savant Instruments, Farmingdale, NY).

### Diagnosis

The hemolysin protein as obtained above was conjugated to KLH as a carrier, using glutaraldehyde chemistry, linking amines of the protein and the carrier. This preparation was injected into animals as an immunogen. However, fungal hemolysins proteins need not be conjugated for use in a vaccine.

Hemolysin was linked to agarose using a cyanogen bromide method and loaded at 1.2 mg of hemolysin peptide/15 ml gel to produce immunosorbent.

The hyperimmune serum of rabbits immunized with the KLH-protein was processed over the above immunosorbent to capture antibodies specific for the fungal hemolysin protein. The antibodies obtained by this procedure were provided as affinity-purified antibodies.

An ELISA assay was conducted by coating peptide onto microliter wells and reacting the peptide with dilutions of antibody or anti-hemolysin/ALP, then with Gxrabbit IgG (h&1)/HRP (not for ALP conjugate) followed by TMB/H₂O₂ or BCIP substrate. The following table indicates the dilutions of the antibody solutions that produced an OD of 1.0 in this assay:

| Specificity Antibody | Antibody Prep | Antisera | Dilution A.P. Antibody |
|---|---|---|---|
| Hemolysin | 7980/2 | 1/41,000 | 1/27,160 |

These antibodies to fungal hemolysin can be used in a conventional immunoassay such as an ELISA to determine of one has been exposed to strains of fungi which produce hemolysin. The hemolysin protein itself can be used to determine if one has produced antibodies in response to exposure to the fungus.

The present invention thus provides a method to determine if a human or other animal has been exposed to a hemolysin-producing fungus such as *Stacybotrys chartarum.* By assaying samples from a human or other animal for antibodies to a hemolysin-producing fungus, it is now possible to determine if the human or other animal has been exposed to such a fungus.

Exposure to low levels of hemolysins can lead to potassium ion depletion in monocytes, which can lead to activation of interleukin-converting enzyme. This in turn can lead to rapid and massive release of mature IL-beta. In addition, T-lymphocytes that leak potassium ions undergo programmed cell death (apoptosis) For this reason, it is critical to identify the source of the hemolysin in a human or other animal which may have been exposed to a hemolysin-producing fungus and immediately begin appropriate treatment.

The method of the present invention is useful for assaying for exposure to or for the presence of any hemolysin-producing fungal strain. Some nonlimiting examples of these fungal strains are *Stacybotrys chartarum, Aspergillus fumigasus, Candida albicans* and *Penicillium chysogenum.*

### Screening Fungi

Many fungi are present in buildings such as offices, homes, schools, warehouses, etc., but not all fungi adversely affect humans. To determine if a building holds fungi which are problematic, i.e., produce hemolysin, a strain of a fungus obtained from the building is grown in a synthetic medium such as tryptic soy broth at a temperature at which the fungus can be made to grow, generally about 37°C. The culture filtrate is then applied to a plate, such as a 5% sheep red blood cell blood agar plate. If the filtrate is shown to be hemolytic, the strain is problematic and may pose a health risk.

Once a building has been found to contain problematic fungi, the building is treated to remove or destroy the fungi. The screening can then be repeated to ensure that the problematic fungi have been eliminated from the site.

### Vaccine

Because antibodies are produced against fungal hemolysin, the protein or a fragment of the protein can be used to prepare a vaccine. Individuals at risk to fungal exposure can then be inoculated with such a vaccine to protect them from fungal infection.

Vaccines can be prepared with the complete hemolysin protein as well as any fragment, functional derivative, variant, analog, or chemical derivative of the protein that substantially retains its above-described biological activity. A "biologically active" portion of any protein herein can also include fragments that retain biological activities such as immunogenicity and immunoreactivity, as can be determined by standard methods known in the art. A "homiletical active portion" of a protein retains the function of hemolysis, as can be readily tested by convention methods.

An immunoreactive fragment, variant, analog, functional derivative, or chemical derivative of a fungal hemolytic protein is defined as an amino acid sequence of at least approximately five consecutive amino acids derived from the protein's amino acid sequence. These fragments can be generated, for example, by mechanical or chemical disruption of the complete protein or, as another example, they can be recombinant proteins obtained by cloning nucleic acids encoding the polypeptide in an expression system capable of producing the protein or fragments thereof. The activity of these fragments can be determined using conventional techniques.

The polypeptide fragments of the present invention can also be recombinant peptides obtained by cloning nucleic acids and encoding the polypeptide in an expression system capable of producing the antigenic polypeptide or fragments thereof.

The present invention-also provides an immunogenic amount of a fungal hemolysin protein in a pharmaceutically acceptable carrier. An immunogenic amount can be readily determined by standard methods for the specific subject to which it is to be administered. Once the amino acid sequence of each protein is deduced from the DNA sequence, it is possible to synthesize, using standard peptide synthesis techniques and/or recombinant techniques, peptide fragments that are homologous to immunoreactive regions of the protein and to modify these fragments by inclusion, deletion or modification of particular amino acid residues in the derived sequencer. Thus, synthesis or purification of an extremely large number of peptides derived from the original protein sequence is possible.

The amino acid sequences of the present polypeptides can contain an immunoreactive portion attached to sequences designed to provide for some additional property, such as solubility. Furthermore, the amino acid sequences can include sequences in which one or more amino acids have been substituted with another amino acid to provide for some additional property, such as to remove or add amino acids capable of disulfide bonding, to increase is biolongevity, alter enzymatic acidity, or later interactions with gastric acidity. In any case, the final peptide must possess the bioactive property such as hemolysin regulation, hemolysis, immunoreactivity, immunogenicity, etc.

The purified polypeptide fragments thus obtained can be tested to determine their immunogenicity and specificity. Briefly, various concentrations of a putative immunogenically specific fragment are prepared and administered to an anima, and the immunological response (e.g., the production of antibodies or cell mediated immunity) of an animal to each concentration is determined. The amounts of antigen administered depend on the subject, the condition of the subject, the size of the subject, etc. Thereafter, an animal so inoculated with the antigen can be exposure to the fungus to test the potential vaccine effect of the specific immunogenic fragment. The specificity of a putative immunogenic fragment can be ascertained by testing sera, other fluids, or lymphocytes from the inoculated animal for cross reactivity with other closely related fungi.

Polynucleotides encoding a variant polypeptide may include sequences that facilitate transcription (expression sequences) and translation of the coding sequences such that the encoded polypeptide product is produced. Construction of such polynucleotides is well known in the art. For example, such polynucleotides can include a promoter, a transcription termination site (polyadenylation site in eukaryotic expression hosts), a ribosome binding site, and, optionally, an enhanced or use in eukaryotic expression hosts, and, optionally sequences necessary for replication of a vector.

An antibody which specifically binds an antigenic portion of the protein is also provided for each fungal hemolytic protein. The antibodies can specifically bind a unique epitope of the antigen, or they can bind epitopes of other organisms. The term "specifically bind" means an antibody specifically binding a protein which does not substantially cross react with any antigen other than the hemolysin fungal protein, such that the intended antigen can be detected.

Antibodies can be made by methods well known in the art, such as described in Harlow and Lane, Antibodies, A Laboratory Manual, Cold Spring Harbor Laboratory, Cold Spring Harbor, NY (1988). Briefly, purified protein, or an antigenic fragment thereof, is injected into an animal in an amount and in intervals sufficient to elicit an immune response. Polyclonal antibodies can be purified directly by passing serum collected from the animal through a column to which non-hemolysin regulatory proteins or non-hemolysin proteins prepared from the same expression system have been bound, monoclonal antibodies can also be produced by obtaining spleen cells from the animal. The cells are then fused with an immortal cell line and screened for antibody secretion. The antibodies can be used to screen DNA clone libraries for cells secreting the antigen. Those positive clones can then be sequenced if desired (cf. Kelly et al., Bio/Technology 10:163-167 (1992); Bebbington et al., Bio/Technology 10:169-175 (1992).

The antibody can be bound to a substrate or labeled with a detectable moiety, or both bound and labeled. The detectable moieties include, for example, fluorescent, enzymatic, and radioactive markers.

Vaccines are produced from an immunogenic amount of the fungal hemolysin protein and a pharmaceutically acceptable carrier. This composition can include the entire antigen, a functional fragment of the antigen, or an epitope specific to the antigen. The antigen can also be potentially cross-reactive with antibodies to other antigens. The composition can be used to prevent fungal infections by fungi which produce hemolysin.

Immunogenic amounts of the antigen can be determined using standard procedures. Briefly, various concentrations of a putative specific immunoreactive epitope are prepared, administered to an animal, and the immunological response (e.g., the production of antibodies) of the animal to each concentration is determined.

The pharmaceutically acceptable carrier can comprise saline or other suitable carriers, as described in Arnon, ED, Synthetic Vaccines I: 83-92, CRC Press, Inc., Boca Raton, FL, 1987. A carrier can be used with the antigen or fragment thereof. An adjuvant can also be a part of the carrier of the antigen, in which case the adjuvant is selected by standard criteria based on the antigen used, the mode of administration, and the subject, Arnon, Ed., 1987. Methods of administration can be by oral or sublingual means, or by injection, depending upon the particular subject to whom it is administered.

With respect to the hemolytic proteins per se, their antigens and antibodies, the entire protein (i.e., protein, antigen, or antibody) or a functional derivative thereof can be used. By "functional derivative" is meant a fragment, variant, analog, or chemical derivative of the protein of interest. A functional derivative retains at least a portion of the amino acid sequence of the protein of interest, which permits its use in vaccines, screening, and pharmaceuticals according to the present invention. This specificity can readily be quantified by determining the IC₅₀ according to Piersbacher et al., U.S. Patent No. 5,648,330, the entire contents of which are hereby incorporated by reference.

A "fragment" of the protein refers to any subset of the molecule, i.e., a shorter peptide. Fragments of interest, of course, are those which have high specificity to hemolysin, or to an antigen or antibody thereof, as the case may be.

A "variant" of the protein of the present invention refers to a molecule which is substantially similar to either the entire peptide or a fragment thereof. Variant peptides may be conveniently prepared by direct chemical synthesis of the variant peptide, using methods well known in the art.

Alternatively, amino acid sequence variants of the proteins of the present invention can be prepared by mutations in the DNAs which encode the proteins of interest. These variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame, and preferably will not create complementary regions that could produce secondary mRNA structure (*cf*,. European Patent Publication No. EP 75,444).

At the genetic level, these variants ordinarily are prepared by site-directed mutagenesis (as exemplified by Adelman et al., DNA 2:183(1983) of nucleotides in the DNA encoding the peptide molecule, thereby producing DNA encoding the variant, and thereafter expressing the DNA in recombinant cell culture. The variants typically exhibit the same qualitative biological activity as the nonvariant peptide.

An "analog" of the proteins of the present invention refers to a non-natural molecule which is substantially similar to either the entire molecule or to an active fragment thereof.

A "chemical derivative" of a protein according to the present invention contains additional chemical moieties not normally part of the protein's amino acid sequence, covalent modifications of the amino acid sequence are included within the scope of this invention. Such modifications may be introduced into the proteins by reacting targeted amino residues from the peptide with an organic derivatizing agent that is capable of reacting with selected side chains or terminal residues.

The types of substitutions which may be made in proteins of the present invention may be based on analysis of the frequencies of amino acid changes between a homologous protein of different species. Based upon such analysis, conservative substitutions may be defined herein as exchanges within one of the following five groups:
I. Small, aliphatic, nonpolar or slightly polar residues:
   Ala, Ser, Thr, Pro, Gly
II. Polar, negatively charged residues and their amides:
   Asp, Asn, Glu, Gln;
III. Polar, positively charged residues:
   His, Arg, Lys;
IV. Large, aliphatic nonpolar residues:
   Met, Leu, Ile, Val, Cys;
V. Large aromatic residues:
   Phe, Trp, Try.

With the following groups, the following substitutions are considered to be "highly conservative:"
Asp/Glu
His/Arg/Lys
Phe/Tyr/Trp
Met/Leu/Ile/Val
a "fragment" of an antigen.

### Pharmaceuticals

Hemolysins have unique membrane interactions in which they create pores in membranes. This characteristic can be used to selectively kill certain cells, such as cancer cells. In this case, the fungal hemolysin is attached to an antibody to the cancer cell. When the antibody reaches the cancerous cell, the hemolysin selectively kills the cancer cells by creating pores in the membrane of the cancer cells.

Additionally, fungal hemolysins can help large molecules pass through the blood-brain barrier by creating pores in the cells which form the blood-brain barrier. Many pharmaceutical compounds are so large that they cannot arrive at the brain because they are unable to cross the blood-brain barrier. By combining such a molecule with a pore forming hemolysin, such as stachylysin, the drug can be delivered to the proper location.

Additionally, fungal hemolysins can be used in antibacterial and antifungal preparations. The fungal hemolysin is incorporated in a suitable carrier such as water or organic solvent, cream, lotion, or the like. Surfactants, perfumes, coloring agents, and the like may optionally be added.

By applying these preparations to a surface, such as skin, walls, kitchen counters, bathroom fixtures, etc., an antimicrobial or antifungal effect is created.

The foregoing description of the specific embodiments will so fully reveal the general nature of the invention that others can, by applying current knowledge, readily modify and/or adapt for various applications such specific embodiments without undue experimentation and without departing from the generic concept. Therefore, such adaptations and modifications should and are intended to be comprehended within the meaning and range of equivalents of the disclosed embodiments. It is to be understood that the phraseology or terminology employed herein is for the purpose of description and not of limitation. The means and materials for carrying our various disclosed functions may take a variety of alternative forms without departing from the invention. Thus, the expressions "means to..." and "means for..." as may be found in the specification above and/or in the claims below, followed by a functional statement, are intended to define and cover whatever structural, physical, chemical, or electrical element or structures which may now or in the future exist for carrying out the recited function, whether or not precisely equivalent to the embodiment or embodiments disclosed in the specification above; and it is intended that such expressions be given their broadest interpretation.

## Claims

1. Isolated fungal hemolysin or active fragments thereof obtained from *Stachybotrys chartarum.*

2. Antibodies to fungal hemolysin or active fragments thereof.

3. A method for determining if a mammal has been exposed to a hemolysin-producing fungus comprising contacting a sample from said mammal with labeled antibodies to fungal hemolysin or active fragments thereof and detecting the label to determine the presence of antigens to fungal hemolysin.

4. The method according to claim 3 wherein the label is selected from the group consisting of enzyme, radioactive, chemiluminescent, and fluorescent labels.

5. The method according to claim 3 wherein the fungus is selected from the group consisting of *Stachybotrys chartarum, Aspergillus fumigatus, Candida albicans, and Penicillium chrysogenum.*

6. A method for isolating fungal hemolysin comprising culturing a strain of fungus on a synthetic medium, removing cells and debris from the culture to recover supernatant, and isolating homiletical active fractions.

7. The method according to claim 6 wherein the strain of fungus is cultured on tryptic soy broth.

8. The method according to claim 6 wherein the strain of fungus is *Stachybotrys chartarum.*

9. The method according to claim 6 wherein the homiletical active fractions are isolated by gel filtration.

10. A composition for treating cancer cells comprising fungal hemolysin conjugated to an antibody for the cancer cell.

11. A vaccine to protect against infection by hemolysin producing fungi comprising an antigen or an active fragment, derivative, analog, or variant thereof to fungal hemolysin and a pharmaceutically acceptable carrier.

12. The vaccine according to claim 11 wherein the hemolysin producing fungi are selected from the group consisting of *Stachybotrys chartarum, Aspergillus fumigatus, Candida albicans,* and *Penicillium chrysogenum.*

13. A method for altering immune function in a mammal in need thereof comprising administering to said mammal an effective amount of a fungal hemolysin to alter immune function.

14. The method according to claim 13 wherein the fungal hemolysins is obtained from a fungus selected from the group consisting of *Stachybotrys chartarum, Aspergillus fumigatus, Candida albicans,* and *Penicillium chrysogenum.*

15. A method for treating cancer comprising conjugating a fungal hemolysin to an antibody to the cancer cell and administering an effective amount of the conjugate to a patient in need thereof.

16. A composition for administration across the blood-brain barrier comprising a combination of a pore-forming fungal hemolysin and at least one pharmaceutically effective compound.

17. An antibacterial composition comprising an antibacterial amount of a fungal hemolysin.

18. An antifungal composition comprising an antifungal amount of a fungal hemolysin.
